# EUROPÄISCHE PATENTSCHRIFT

(11) **EP 2 653 116 B1**
(45) Veröffentlichungstag und Bekanntmachung des Hinweises auf die Patenterteilung: **27.12.2017**
(21) Anmeldenummer: 13001765.0
(22) Anmeldetag: 06.04.2013
(51) Int. Cl.: A61B 17/29, A61B 17/00

(54) **Handhabungseinrichtung für ein medizinisches Instrument**
Handling device for a medical instrument
Dispositif de manipulation d'un instrument médical

(30) Priorität: 18.04.2012 DE 102012007649
(43) Veröffentlichungstag der Anmeldung: 23.10.2013
(73) Patentinhaber: Karl Storz GmbH & Co. KG, 78532 Tuttlingen (DE)
(72) Erfinder: Kärcher, Daniel, 78532 Tuttlingen (DE); Stefan, Jochen, 78532 Tuttlingen (DE)

(56) Entgegenhaltungen:
- DE-A1- 10 236 274
- DE-A1- 19 505 032
- DE-A1-102004 049 243
- DE-B3- 10 357 105
- US-A- 5 505 737
- US-A- 6 139 214
- US-A1- 2009 069 842
- US-A1- 2009 171 147

## Beschreibung

Die vorliegende Erfindung ist auf eine Handhabungseinrichtung zur mechanischen Verbindung mit dem proximalen Ende eines Schafts, zur Bildung eines medizinischen Instruments und auf ein medizinisches Instrument mit einer solchen Handhabungseinrichtung bezogen.

Die Erwartungen an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende, bei denen das Werkzeug um die Längsachse des Schafts rotierbar ist. Die Greif- oder Schneidefunktion und die Rotation des Werkzeugs sind beispielsweise mittels einer einzigen Übertragungsstange steuerbar, die Längskräfte und Drehmomente überträgt. Hinzu kommt in neuerer Zeit eine Abwinkelbarkeit des Schafts proximal des Werkzeugs, zu deren Steuerung ein zweites Übertragungselement im Schaft des medizinischen Instruments vorgesehen sein kann, beispielsweise eine zweite Übertragungsstange.

Bei wiederverwendbaren medizinischen Instrumenten ist für die Reinigung eine möglichst weitgehende Zerlegbarkeit erforderlich. Insbesondere ist bei vielen medizinischen Instrumenten die Handhabungseinrichtung vom proximalen Ende des Schafts trennbar. Dabei kann beispielsweise eine Rastverbindung zwischen dem proximalen Ende des Schafts bzw. des Außenschafts einerseits und der Handhabungseinrichtung andererseits durch manuellen Druck auf einen Entriegelungsknopf oder Betätigung einer anderen Betätigungseinrichtung entriegelt werden. Bei von der Anmelderin hergestellten und unter der Bezeichnung "Clickline" vertriebenen medizinischen Instrumenten gelangen das proximale Ende der Übertragungsstange und ein mit dieser gekoppelter Hebel der Halteeinrichtung beim Herausziehen des Schafts nach distal aus der Handhabungseinrichtung in Positionen, in denen sie nicht mehr miteinander gekoppelt sind. Die Kopplung zwischen dem proximalen Ende der Übertragungsstange und der Handhabungseinrichtung bzw. einem Hebel an der Handhabungseinrichtung ist somit lösbar, wenn die Kopplung zwischen dem proximalen Ende des Schafts und der Handhabungseinrichtung gelöst ist.

Die von "Clickline"-Produkten bekannte Lösbarkeit bzw. Trennbarkeit der proximalen Enden des Außenschafts und der Übertragungsstange einerseits von der Handhabungseinrichtung andererseits kann jedoch auf manch andere medizinische Instrumente nicht oder nicht ohne Weiteres übertragen werden. Eine Alternative besteht darin, für jede Kopplung eine separate Betätigungseinrichtung vorzusehen. Im Fall eines medizinischen Instruments mit einem Außenschaft und zwei Übertragungsstangen oder anderen Übertragungseinrichtungen sind also drei Betätigungseinrichtungen vorzusehen, mittels derer manueller Betätigung jeweils eine Kopplung zwischen dem proximalen Ende des Außenschafts bzw. einer Übertragungsstange einerseits und der Handhabungseinrichtung andererseits lösbar ist.

In US 2009/0069842 A1 ist ein chirurgisches Instrument für minimalinvasive Verfahren beschrieben. Ein Schaft und eine wiederverwendbare Handhabe können voneinander getrennt werden. Ein Schaftverbinder am proximalen Ende des Schafts weist eine umlaufende Nut auf. An der Handhabe ist eine Schaftverriegelungseinrichtung mit einem Bauteil mit einem halbkreisförmigen Rand vorgesehen. Zur Verriegelung des Schafts an der Handhabe greift der Rand des Bauteils in die Nut am proximalen Ende des Schafts ein. Das proximale Ende eines Seils zur Betätigung eines Effektors am distalen Ende des Schafts weist einen Ansatz ("lug") auf. Der Ansatz kann von einer Kabelkopplungseinrichtung mit vier elastisch auslenkbaren Backen hintergriffen und gehalten werden, wenn die Backen von einer konischen Oberfläche zusammengedrückt werden. Durch Betätigen eines flügel- oder hebelförmigen Bauteils können die Verbindungen der Handhabe mit dem Schaft und dem Ansatz getrennt werden. Dazu ist das hebelförmige Bauteil durch ein Pleuel mit dem Bauteil und durch eine Gabel mit Nocken gekoppelt. Die Nocken können die konische Oberfläche gegen die Kraft einer Feder in eine Position verschieben, in der sie die Backen nicht mehr zusammen hält und der Ansatz freigegeben ist. Diese Wirkung haben die Nocken jedoch nur, wenn die Kabelkopplungseinrichtung sich in einer distalen Position befindet. Auch bei weiteren beschriebenen Ausführungsformen ist eine Entriegelung nur in einer (distalen) Position möglich.

In US 2009/0171147 A1 sind ähnliche chirurgische Instrument beschrieben, bei denen eine Entriegelung einer Verbindung zwischen Handhabe einerseits und Ansatz am proximalen Ende eines Seils andererseits nur in einer distalen Position möglich ist.

In DE 102 36 274 A1 ist ein modulares Zangensystem mit einem Griffteil und einem abnehmbaren Zangenschaft beschrieben. Der Zangenschaft weist am proximalen Ende nach radial außen gerichtete und elastisch auslenkbare Rasthaken auf, die hinter einen nach radial innen gerichteten ringförmigen Rastvorsprung in einer Aufnahmeöffnung am Griffteil greifen können, um den Zangenschaft formschlüssig mit dem Griffteil zu verbinden. Eine im Zangenschaft angeordnete Betätigungsstange weist am proximalen Ende nach radial innen gerichtete und elastisch auslenkbare Rasthaken auf, die hinter ein nach radial außen gerichtetes ringförmiges Rastteil greifen können, um die Betätigungsstange mit einer Handhabe zu koppeln. Durch Druck auf einen Betätigungsknopf kann gegen die Kraft einer Druckfeder ein Betätigungskörper nach distal verschoben werden, der mit einem ringförmigen Abschnitt die Rasthaken am Zangenschaft nach radial innen auslenkt und mit einem Zapfen die Rasthaken an der Betätigungsstange nach radial außen auslenkt und so eine Trennung des Zangenschafts und der Betätigungsstange von dem Griffteil ermöglicht. Da die Auslenkung der Rasthaken an der Betätigungsstange mittels des Betätigungskörpers nur in einer proximalen Position des Rastteils und der Betätigungsstange möglich ist, ist am Betätigungsknopf eine Feder vorgesehen, um die Handhabe so zu bewegen, dass das Rastteil und die Betätigungsstange nach proximal verschoben werden.

In US 6,139,214 ist eine Schnellkupplung zur lösbaren Verbindung eines Handhabungsteils eines chirurgischen Instruments mit einem Einweg-Arbeitsteil beschrieben. Im Handhabungsteil ist ein Verriegelungsmechanismus aus zwei jeweils ringförmigen Baugliedern vorgesehen. Jedes Bauglied weist jeweils an einer Seite eine manuell betätigbare Oberfläche und an einer gegenüberliegenden Seite ein Verriegelungselement auf. Das Arbeitsteil kann in dem Handhabungsteil formschlüssig gehalten werden, indem die Verriegelungselemente eine Verriegelungslamelle an dem Arbeitsteil hintergreifen. Durch manuellen Druck auf die Oberflächen können die Bauglieder gegen die Kraft einer Feder so weit verschoben werden, dass die formschlüssige Verbindung gelöst wird.

In US 5,505,737 ist eine Schnellkupplung an einem chirurgischen Instrument zwischen einem fluidisch angetriebenen Motor und einem rotierbaren Dissektionswerkzeug beschrieben. In radialer Richtung bewegbare Kugeln an einem mit dem Motor gekoppelten Bereich werden durch eine Feder vermittels einer konischen Oberfläche in Ausnehmungen am Schaft des Dissektionswerkzeugs gedrückt, um den Schaft mit dem Motor zu koppeln. Durch axiales Verschieben eines Kragens kann die konische Oberfläche gegen die Kraft der Feder verschoben werden, so dass die Kugeln die Ausnehmungen im Schaft verlassen und der Schaft vom Motor getrennt werden kann.

DE 195 05032 A1 zeigt ein chirurgisches Rohrschaftinstrument mit Handhabe, bei dem der Schaft lösbar durch eine Halteeinrichtung gehalten bzw. gelöst ist, was durch eine Betätigungseinrichtung an der Halteeinrichtung in verschiedenen Positionen der Halteeinrichtung bewirkt werden kann.

Eine Aufgabe der vorliegenden Erfindung besteht darin, eine verbesserte Handhabungseinrichtung für ein medizinisches Instrument und ein verbessertes medizinisches Instrument zu schaffen.

Diese Aufgabe wird durch den Gegenstand des unabhängigen Anspruchs gelöst.

Weiterbildungen sind in den abhängigen Ansprüchen angegeben.

Ausführungsbeispiele der vorliegenden Erfindung beruhen auf der Idee, an einer Handhabungseinrichtung eine Mehrzahl von Halteeinrichtungen zum lösbaren Halten von jeweils einem proximalen Ende eines Schafts oder einer Übertragungseinrichtung eines medizinischen Instruments derart mit einer Betätigungseinrichtung zu koppeln, dass durch manuelle Betätigung der Betätigungseinrichtung alle Halteeinrichtungen gelöst werden können. Eine so ausgestattete Handhabungseinrichtung kann mit einem einzigen Handgriff und damit besonders schnell und einfach vollständig vom Schaft und einer oder mehreren Übertragungseinrichtungen des Schafts getrennt werden. Die Zerlegung des medizinischen Instruments kann damit deutlich geringere Anforderungen an medizinisches Personal stellen, als wenn mehrere Betätigungseinrichtungen gleichzeitig oder nacheinander oder gar in einer vorbestimmten Reihenfolge betätigt werden müssten. Damit sinkt auch das Risiko einer Beschädigung des medizinischen Instruments durch eine falsche Handhabung bei der Zerlegung.

Ausführungsbeispiele der vorliegenden Erfindung beruhen ferner auf der Idee, an einer Handhabungseinrichtung eine manuell betätigbare Betätigungseinrichtung derart mit einer bewegbaren Halteeinrichtung zum lösbaren Halten des proximalen Endes eines Schafts oder einer Übertragungseinrichtung mechanisch zu koppeln, dass unabhängig von der relativen Position von Halteeinrichtung und Betätigungseinrichtung eine Betätigung der Betätigungseinrichtung ein Lösen der Halteeinrichtung bewirkt. Dies kann nach einer Verwendung eines medizinischen Instruments und vor dessen Reinigung oder Umkonfigurierung eine Trennung der Handhabungseinrichtung von einem Schaft vereinfachen, indem beispielsweise nicht erst ein Werkzeug in eine bestimmte Position gebracht werden muss. Dadurch können der Schulungsaufwand für Personal und das Risiko einer Beschädigung aufgrund falscher Handhabung gesenkt werden. Ferner kann der Zeitaufwand für die Zerlegung eines medizinischen Instruments reduziert werden.

Eine Handhabungseinrichtung zur mechanischen Verbindung mit dem proximalen Ende eines Schafts, zur Bildung eines medizinischen Instruments, umfasst eine Mehrzahl von Halteeinrichtungen, wobei jede einzelne der Mehrzahl von Halteeinrichtungen zum lösbaren Halten des proximalen Endes eines Schafts oder des proximalen Endes einer Übertragungseinrichtung zum Übertragen zumindest entweder einer Kraft oder eines Drehmoments in dem Schaft ausgebildet ist, eine manuell betätigbare Betätigungseinrichtung und eine Kopplungseinrichtung zur mechanischen Kopplung der Betätigungseinrichtung mit der Mehrzahl von Halteeinrichtungen derart, dass eine manuelle Betätigung der Betätigungseinrichtung ein Lösen der Mehrzahl von Halteeinrichtungen bewirkt.

Die Handhabungseinrichtung ist insbesondere vorgesehen und ausgebildet, um mit einem Schaft lösbar mechanisch verbunden zu werden, der in einem Außenschaft eine oder mehrere Übertragungseinrichtungen in Form von einer oder mehreren Übertragungsstangen und/oder in Form eines Innenschafts aufweist. Die eine oder mehreren Übertragungseinrichtungen sind relativ zu einander und relativ zum Innenschaft in Richtung parallel zur Längsachse des Außenschafts und damit insbesondere auch parallel zu den Längsachsen der Übertragungseinrichtungen verschiebbar und/oder um die Längsachse rotierbar, um jeweils die Übertragung einer Kraft und/oder eines Drehmoments zum distalen Ende des Schafts zu ermöglichen. Insbesondere ist die Handhabungseinrichtung zur lösbaren mechanischen Verbindung mit einem Außenschaft, einem Innenschaft und einer Übertragungsstange, die koaxial angeordnet sind, ausgebildet.

Jede Halteeinrichtung ist insbesondere ausgebildet, um Kräfte in Richtung parallel zur Längsachse des Schafts und damit insbesondere auch in Richtung parallel zur Längsachse einer Übertragungseinrichtung auf deren proximales Ende zu übertragen. Jede Halteeinrichtung kann ferner dazu ausgebildet sein, ein Drehmoment auf das proximale Ende des zugeordneten Schafts bzw. auf das proximale Ende der zugeordneten Übertragungseinrichtung zu übertragen. Dazu weist die Halteeinrichtung insbesondere eine Ausnehmung für das proximale Ende des Schafts bzw. der Übertragungseinrichtung auf, die einen nicht-rotationssymmetrischen und zum proximalen Ende des Schafts bzw. der Übertragungseinrichtung korrespondierenden Querschnitt hat.

Jede einzelne Halteeinrichtung weist insbesondere einen haltenden bzw. geschlossenen bzw. verriegelten Zustand und einen gelösten bzw. offenen bzw. entriegelten Zustand auf. Im haltenden Zustand kann die Halteeinrichtung das proximale Ende eines Schafts oder einer Übertragungseinrichtung halten bzw. mit dem proximalen Ende mechanisch verbunden sein, und zwar insbesondere form-, kraft- oder reibschlüssig. Die Halteeinrichtung kann ausgebildet sein, um auch in Abwesenheit eines proximalen Endes eines Schafts oder einer Übertragungseinrichtung einen Zustand einzunehmen, der dem haltenden Zustand entspricht. Im gelösten Zustand kann ein proximales Ende eines Schafts oder einer Übertragungseinrichtung von der Halteeinrichtung getrennt werden. Die Halteeinrichtung ist insbesondere ausgebildet, um nur im gelösten Zustand ein proximales Ende eines Schafts oder einer Übertragungseinrichtung in die Halteinrichtung aufzunehmen. Dabei kann die Halteeinrichtung ausgebildet sein, um beim Einführen eines proximalen Endes eines Schafts oder einer Übertragungseinrichtung selbsttätig vorübergehend in den gelösten Zustand überzugehen

Jede einzelne Halteeinrichtung umfasst insbesondere einen Riegel, einen Schieber, eine Raste oder eine Klinke zum formschlüssigen Halten bzw. zur formschlüssigen mechanischen Verbindung mit dem proximalen Ende eines Schafts oder dem proximalen Ende einer Übertragungseinrichtung. Dabei greift die Halteeinrichtung oder deren Riegel, Schieber, Raste oder Klinke insbesondere in eine korrespondierende Ausnehmung an dem proximalen Ende des Schafts oder der Übertragungseinrichtung ein. Der Riegel, der Schieber, die Raste bzw. die Klinke ist insbesondere in einer Richtung senkrecht zur Längsachse des proximalen Endes des Schafts bzw. der Übertragungseinrichtung bewegbar. In einer Halte- bzw. Verriegelungsposition hält der Riegel, der Schieber, die Raste bzw. die Klinke das proximale Ende des Schafts bzw. der Übertragungseinrichtung formschlüssig an oder in der Halteeinrichtung. In einer Löseposition des Riegels, des Schiebers, der Raste bzw. der Klinke ist das proximale Ende des Schafts bzw. der Übertragungseinrichtung freigegeben und kann von der Halteeinrichtung getrennt werden. Der Riegel, der Schieber, die Raste bzw. die Klinke kann durch eine Feder oder ein anderes elastisches Element in die Halteposition bewegt werden.

Alternativ kann eine Halteeinrichtung zum kraft- bzw. reibschlüssigen Halten eine proximalen Endes eines Schafts oder einer Übertragungseinrichtung ausgebildet sein.

Die manuell betätigbare Betätigungseinrichtung umfasst insbesondere einen Druckknopf, einen Schieber oder einen Hebel, der manuell in einer Richtung parallel zur Längsachse eines mit der Handhabungseinrichtung zu verbindenden Schafts oder senkrecht dazu bewegbar ist. Die Betätigungseinrichtung ist an der Handhabungseinrichtung insbesondere so angeordnet, dass einerseits mit einer Hand, die die Handhabungseinrichtung hält, die Betätigungseinrichtung ohne Weiteres betätigt werden kann, und dass andererseits eine unbeabsichtigte Betätigung unwahrscheinlich oder ausgeschlossen ist.

Die Kopplungseinrichtung koppelt die Betätigungseinrichtung mit der Mehrzahl von Halteeinrichtungen insbesondere derart, dass bei einer vollständigen manuellen Betätigung der Betätigungseinrichtung (beispielsweise Drücken eines Druckknopfs oder Ziehen an einem Schieber bis zu einem Anschlag) alle Halteeinrichtungen unmittelbar durch Einwirkung der Kopplungseinrichtung gelöst werden. Die unmittelbare mechanische Kopplung der Mehrzahl von Halteeinrichtungen mit der Betätigungseinrichtung mittels der Kopplungseinrichtung bedeutet insbesondere nicht, dass infolge des Lösens einer ersten der Mehrzahl von Halteeinrichtungen eine Veränderung an dem medizinischen Instrument lediglich ermöglicht wird, die das Lösen einer zweiten der Mehrzahl von Halteeinrichtungen bewirkt oder ermöglicht. Darin unterscheidet sich die vorliegende Erfindung von den unter der Bezeichnung "Clickline" vertrieberien Produkten der Anmelderin. Dies schließt jedoch nicht aus, dass zusätzlich zu der Mehrzahl von Halteeinrichtungen an der Handhabungseinrichtung eine oder mehrere weitere Halteeinrichtungen vorgesehen sind, die im genannten Sinn lediglich mittelbar gelöst werden. Ebenso wenig ist ausgeschlossen, dass die Mehrzahl von Halteeinrichtungen nicht vollständig gleichzeitig gelöst wird, dass also (insbesondere bei einer langsamen Betätigung der Betätigungseinrichtung) die Mehrzahl von Halteeinrichtungen zumindest teilweise nacheinander gelöst wird.

Bei einer Handhabungseinrichtung, wie sie hier beschrieben ist, sind insbesondere eine erste Halteeinrichtung der Mehrzahl von Halteeinrichtungen ausgebildet, um ein proximales Ende eines Schafts zu halten, und eine zweite Halteeinrichtung der Mehrzahl von Halteeinrichtungen ausgebildet, um ein proximales Ende einer Übertragungseinrichtung zum Übertragen zumindest entweder einer Kraft oder eines Drehmoments zwischen der Handhabungseinrichtung und einem Werkzeug am distalen Ende des Schafts zu halten.

Bei der Handhabungseinrichtung ist insbesondere ferner eine dritte Halteeinrichtung der Mehrzahl von Halteeinrichtungen ausgebildet, um ein proximales Ende einer weiteren Übertragungseinrichtung zum Übertragen zumindest entweder einer Kraft oder eines Drehmoments zu einem Gelenk am Schaft zu halten.

Die Übertragungseinrichtung ist insbesondere eine Übertragungsstange zum Übertragen einer Kraft und eines Drehmoments, die biegeflexibel und torsionssteif ausgebildet sein kann. Die weitere Übertragungseinrichtung umfasst insbesondere einen rohrförmigen oder schlauchartigen Innenschaft, der koaxial in einem ringförmigen Zwischenraum zwischen dem Schaft bzw. Außenschaft einerseits und der Übertragungsstange andererseits angeordnet ist. Der Innenschaft ist insbesondere vollständig oder abschnittsweise starr, biegeflexibel oder abwinkelbar ausgebildet. Der Innenschaft und die Übertragungsstange sind relativ zum Schaft bzw. Außenschaft insbesondere unabhängig voneinander in Längsrichtung spiel- und reibungsarm verschiebbar und um die Längsrichtung bzw. Längsachse rotierbar.

Die Handhabungseinrichtung ermöglicht ein im oben beschriebenen Sinne gleichzeitiges Lösen der mechanischen Verbindung zwischen dem proximalen Ende des Außenschafts und der dem Außenschaft zugeordneten ersten Halteeinrichtung, der Verbindung zwischen dem proximalen Ende der Übertragungsstange und der der Übertragungsstange zugeordneten zweiten Halteeinrichtung und der mechanischen Verbindung zwischen dem Innenschaft und der dem Innenschaft zugeordneten dritten Halteeinrichtung.

Bei einer Handhabungseinrichtung, wie sie hier beschrieben ist, sind insbesondere eine Halteeinrichtung der Mehrzahl von Halteeinrichtungen zusammen mit einem von der Halteeinrichtung gehaltenen proximalen Ende eines Schafts oder einer Übertragungseinrichtung relativ zu der Betätigungseinrichtung bewegbar und die Kopplungseinrichtung ausgebildet, um die Betätigungseinrichtung unabhängig von der Position der bewegbaren Halteeinrichtung relativ zur Betätigungseinrichtung mit der bewegbaren Halteeinrichtung zu koppeln.

Insbesondere sind mehrere oder alle Halteeinrichtungen der Mehrzahl von Halteeinrichtungen jeweils zusammen mit den zugeordneten proximalen Enden eines Schafts oder einer Übertragungseinrichtung relativ zu der Betätigungseinrichtung bewegbar. Mit der Bewegbarkeit einer Halteeinrichtung ist nicht die zum Herstellen einer Verbindung mit dem zugeordneten proximalen Ende eines Schafts oder einer Übertragungseinrichtung oder zum Lösen dieser Verbindung bzw. zum Verriegeln oder Entriegeln der Verbindung vorgesehene und erforderliche Bewegung einer Halteeinrichtung oder eines Teils einer Halteeinrichtung gemeint. Gemeint ist vielmehr jeweils die Bewegbarkeit einer Halteeinrichtung in der (translatorischen oder rotatorischen) Richtung, in der die Halteeinrichtung zusammen mit einem zugeordneten und mechanisch verbundenen proximalen Ende eines Schafts oder einer Übertragungseinrichtung bewegbar ist. Die Position einer bewegbaren Halteeinrichtung umfasst deren Position hinsichtlich translatorischen und/oder rotatorischen Freiheitsgraden.

Eine Handhabungseinrichtung zur mechanischen Verbindung mit dem proximalen Ende eines Schafts, zur Bildung eines medizinischen Instruments, umfasst eine Halteeinrichtung zum lösbaren Halten des proximalen Endes eines Schafts oder des proximalen Endes einer Übertragungseinrichtung zum Übertragen zumindest entweder einer Kraft oder eines Drehmoments, eine manuell betätigbare Betätigungseinrichtung und eine Kopplungseinrichtung zur mechanischen Kopplung der Betätigungseinrichtung mit der Halteeinrichtung derart, dass eine manuelle Betätigung der Betätigungseinrichtung ein Lösen der Halteeinrichtung bewirkt, wobei die Halteeinrichtung zusammen mit einem von der Halteeinrichtung gehaltenen proximalen Ende eines Schafts oder einer Übertragungseinrichtung relativ zu der Betätigungseinrichtung bewegbar ist, und wobei die Kopplungseinrichtung ausgebildet ist, um die Betätigungseinrichtung unabhängig von der Position der bewegbaren Halteeinrichtung relativ zur Betätigungseinrichtung mit der bewegbaren Halteeinrichtung zu koppeln.

Hinsichtlich des medizinischen Instruments, für das die Handhabungseinrichtung vorgesehen ist, hinsichtlich der Ausbildung der Halteeinrichtung, ihrer Funktion und Wirkung, hinsichtlich Betätigungseinrichtung, hinsichtlich der Kopplungseinrichtung und ihrer Funktion und Wirkung gelten die obigen Ausführungen entsprechend.

Bei einer Handhabungseinrichtung mit einer bewegbaren Halteeinrichtung, wie sie hier beschrieben ist, ist insbesondere die bewegbare Halteeinrichtung zusammen mit einem von der bewegbaren Halteeinrichtung gehaltenen proximalen Ende eines Schafts oder einer Übertragungseinrichtung relativ zu der Betätigungseinrichtung parallel zur Längsachse des Schafts bzw. der Übertragungseinrichtung verschiebbar.

Insbesondere ist die bewegbare Halteeinrichtung zusammen mit einem von der bewegbaren Halteeinrichtung gehaltenen proximalen Ende einer Übertragungsstange oder eines Innenschafts parallel zu dessen Längsachse verschiebbar, um beispielsweise eine Übertragung einer Kraft zum Öffnen oder Schließen eines Maulteils am distalen Ende des Schafts oder zum Abwinkeln des Schafts an einem Gelenk zu ermöglichen.

Bei einer Handhabungseinrichtung mit einer bewegbaren Halteeinrichtung, wie sie hier beschrieben ist, ist insbesondere die bewegbare Halteeinrichtung zusammen mit einem von der bewegbaren Halteeinrichtung gehaltenen proximalen Ende eines Schafts oder einer Übertragungseinrichtung relativ zu der Betätigungseinrichtung um eine Längsachse des Schafts bzw. der Übertragungseinrichtung rotierbar.

Beispielsweise ist die bewegbare Halteeinrichtung zusammen mit einem von der bewegbaren Halteeinrichtung gehaltenen proximalen Ende einer Übertragungsstange rotierbar, um eine Übertragung eines Drehmoments und einer Drehbewegung zu einem Werkzeug am distalen Ende des Schafts zu ermöglichen. Beispielsweise ist die bewegbare Halteeinrichtung zusammen mit dem proximalen Ende eines Innenschafts rotierbar, um eine Rotation eines Gelenks, an dem der Schaft abwinkelbar ist, und einer durch das Gelenk definierten Schwenkachse um eine Längsachse des Schafts zu ermöglichen.

Bei einer Handhabungseinrichtung, wie sie hier beschrieben ist, ist insbesondere die Betätigungseinrichtung zur Betätigung in Richtung parallel zur Längsachse eines mit der Handhabungseinrichtung verbundenen Schafts manuell verschiebbar.

Die Betätigungseinrichtung ist beispielsweise in Form einer zylindrischen Hülse ausgebildet, die mit zwei Fingern gefasst und nach proximal gezogen werden kann, um die Halteeinrichtung bzw. die Halteeinrichtungen zu lösen.

Bei einer Handhabungseinrichtung, wie sie hier beschrieben ist, umfasst die Kopplungseinrichtung insbesondere ein Kopplungselement, das in einer ersten Bewegungsrichtung verschiebbar ist, und eine Gleitfläche mit einem Bereich, der nicht parallel zur ersten Bewegungsrichtung ist, um eine Verschiebung des Kopplungselements in der ersten Bewegungsrichtung in eine Bewegung eines Riegels in einer zweiten Bewegungsrichtung, die von der ersten Bewegungsrichtung verschieden ist, umzusetzen.

Das Kopplungselement ist insbesondere identisch oder teilweise identisch oder starr verbunden mit der Betätigungseinrichtung oder derart mit der Betätigungseinrichtung gekoppelt, dass es bei einer manuellen Betätigung der Betätigungseinrichtung in der ersten Bewegungsrichtung bewegt wird. Die erste Bewegungsrichtung ist insbesondere parallel zur Längsachse eines mit der Handhabungseinrichtung verbundenen Schafts.

Die Gleitfläche besitzt aufgrund ihrer Nichtparallelität bzw. Neigung zur ersten Bewegungsrichtung eine Funktion ähnlich der von einem Keil bekannten. Die Gleitfläche kann als konische Fläche ausgebildet sein oder einen konischen Bereich umfassen, um unabhängig von ihrer Winkelposition hinsichtlich einer Rotation um die Symmetrieachse der konischen Fläche zu wirken. Die Gleitfläche kann an dem Kopplungselement vorgesehen sein. Eine Verschiebung des Kopplungselements in der ersten Bewegungsrichtung kann unmittelbar oder mittelbar in eine Bewegung eines Riegels in der zweiten Bewegungsrichtung umgesetzt werden. Eine unmittelbare Umsetzung kann beispielsweise gegeben sein, wenn an der Gleitfläche der Riegel und das Kopplungselement aneinander anliegen, wobei die Gleitfläche an dem Riegel oder an dem Kopplungselement vorgesehen sein kann. Eine mittelbare Umsetzung kann durch weitere Elemente zwischen der Gleitfläche und dem Riegel erfolgen.

Bei einer Handhabungseinrichtung, wie sie hier beschrieben ist, liegt insbesondere der Riegel unmittelbar an der Gleitfläche an. Beispielsweise ist das Kopplungselement identisch oder teilweise identisch mit der hülsenförmigen Betätigungseinrichtung. Die Außenseite der Betätigungseinrichtung ist durch ihre Anordnung und Formgebung (beispielsweise Riffelung) zur manuellen Betätigung ausgebildet. Die Innenseite der hülsenförmigen Betätigungseinrichtung weist eine konische Gleitfläche oder eine Gleitfläche mit konischem Bereich auf, an der ein Ende des Riegels anliegt. Die Symmetrieachse der konischen Gleitfläche oder des konischen Bereichs der Gleitfläche ist insbesondere mit der Längsachse des Schafts identisch. Eine manuelle Bewegung der Betätigungseinrichtung bzw. des Koppelelements parallel zur Symmetrieachse der konischen Gleitfläche kann eine bezogen auf die Längsachse des Schafts radiale Bewegung des Riegels bewirken.

Eine Handhabungseinrichtung mit einem Kopplungselement, wie sie hier beschrieben ist, weist insbesondere ein weiteres Kopplungselement auf, das an dem Kopplungselement anliegt und in der zweiten Bewegungsrichtung bewegbar ist, wobei der Riegel mit dem weiteren Kopplungselement mechanisch gekoppelt ist.

Die Gleitfläche ist insbesondere an dem Kopplungselement vorgesehen und liegt an dem weiteren Kopplungselement an, oder die Gleitfläche ist an dem weiteren Kopplungselement vorgesehen und liegt an dem Kopplungselement an. Ferner können sowohl das Kopplungselement als auch das weitere Kopplungselement je eine Gleitfläche mit einem Bereich, der nicht parallel zur ersten Bewegungsrichtung bzw. zur ersten Bewegungsrichtung geneigt ist, aufweisen, die aneinander anliegen. Das weitere Kopplungselement ist insbesondere in Längsrichtung eines mit der Handhabungseinrichtung zu verbindenden Schafts ausgedehnt (beispielsweise in Form eines plattenförmigen Bauteils) und liegt an einem oder mehreren in der Längsrichtung voneinander beabstandeten Riegeln von einer bzw. mehreren Halteeinrichtung an. Eine derartige Ausgestaltung des weiteren Kopplungselements kann eine Kopplung der Betätigungseinrichtung mit einer oder mehreren in der Längsrichtung verschiebbaren Halteeinrichtungen unabhängig von deren Position bzw. Positionen ermöglichen.

Bei einer Handhabungseinrichtung mit einem weiteren Kopplungselement, wie sie hier beschrieben ist, kann insbesondere entweder das weitere Kopplungselement die Gleitfläche aufweisen oder die Gleitfläche an dem weiteren Kopplungselement gleitend anliegen.

Bei einer Handhabungseinrichtung mit einer bewegbaren Halteeinrichtung und einem weiteren Kopplungselement, wie sie hier beschrieben ist, sind insbesondere die bewegbare Halteeinrichtung zusammen mit einem von der bewegbaren Halteeinrichtung gehaltenen proximalen Ende eines Schafts oder einer Übertragungseinrichtung relativ zu der Betätigungseinrichtung parallel zur Längsachse des Schafts bzw. der Übertragungseinrichtung verschiebbar und das weitere Kopplungselement und die bewegbare Halteeinrichtung ausgebildet, um bei allen Positionen der bewegbaren Halteeinrichtung miteinander mechanisch gekoppelt zu sein.

Insbesondere weist das weitere Kopplungselement eine Anlagefläche auf, die parallel zur Längsachse eines von der bewegbaren Halteeinrichtung gehaltenen proximalen Endes eines Schafts oder einer Übertragungseinrichtung ist, und an der ein Riegel der bewegbaren Halteeinrichtung anliegt.

Bei einer Handhabungseinrichtung, wie sie hier beschrieben ist, ist insbesondere die zweite Bewegungsrichtung zumindest entweder senkrecht zur ersten Bewegungsrichtung oder senkrecht zur Längsachse eines mit der Handhabungseinrichtung verbundenen Schafts.

Ein medizinisches Instrument umfasst eine Handhabungseinrichtung, wie sie hier beschrieben ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Schnittdarstellung eines Teils einer Handhabungseinrichtung;
- Figur 3: eine weitere schematische Schnittdarstellung des Teils der Handhabungseinrichtung aus Figur 2;
- Figur 4: eine weitere schematische Schnittdarstellung des Teils der Handhabungseinrichtung aus den Figuren 2 und 3;
- Figur 5: eine weitere schematische Schnittdarstellung des Teils der Handhabungseinrichtung aus den Figuren 2 bis 4;
- Figur 6: eine weitere schematische Schnittdarstellung des Teils der Handhabungseinrichtung aus den Figuren 2 bis 5;
- Figur 7: eine schematische Schnittdarstellung eines Teils einer weiteren Handhabungseinrichtung.

### Beschreibung der Ausführungsformen

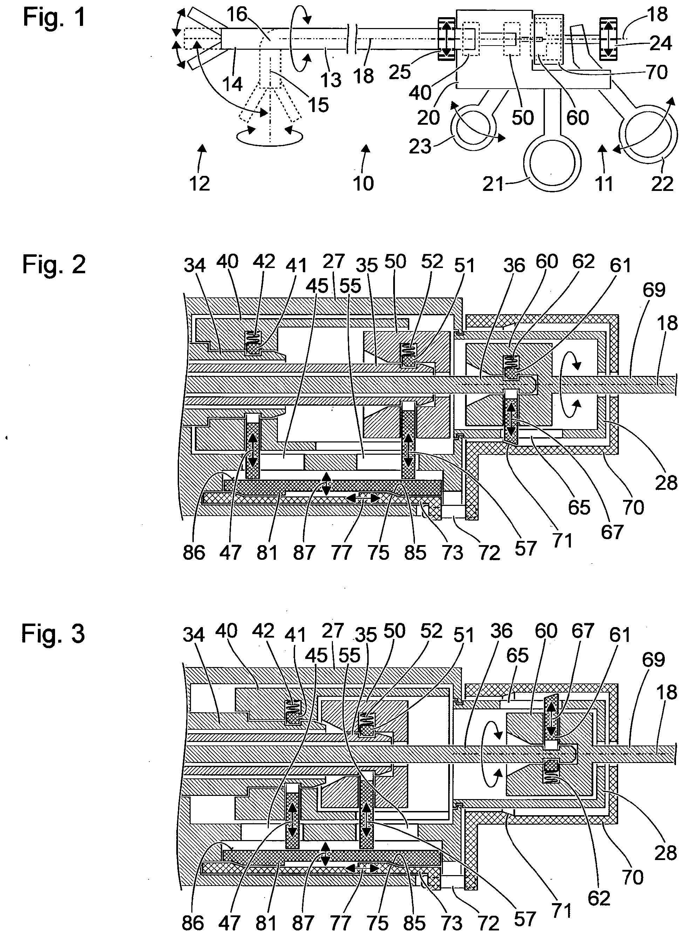

Figur 1 zeigt eine schematische Darstellung eines medizinischen Instruments 10 mit einem proximalen Ende 11 und einem distalen Ende 12. Ein Schaft, der einen Außenschaft 13, einen Innenschaft und eine Übertragungstange umfasst, erstreckt sich bis zum distalen Ende 12 des medizinischen Instruments 10. Am distalen Ende des Außenschafts 13 ist ein Werkzeug 14 vorgesehen, das beispielhaft als Greif- oder Schneidewerkzeug mit zwei bewegbaren Maulteilen angedeutet ist. Proximal des Werkzeugs 14 ist ein Gelenk 16 am Außenschaft 13 vorgesehen.

Eine Reihe von Pfeilen und gestrichelten Konturen deuten Bewegbarkeiten bzw. Freiheitsgrade an. Insbesondere können die beiden Maulteile des Werkzeugs 14 geöffnet bzw. voneinander weg bewegt und geschlossen bzw. aufeinander zu bewegt werden (Schwenkachsen der Maulteile senkrecht zur Zeichenebene der Figur 1); das Werkzeug 14 mit seinen Maulteilen um seine Längsachse 15 rotiert werden; das Werkzeug 14 mit samt seiner Längsachse 15 um eine durch das Gelenk 16 definierte Schwenkachse geschwenkt werden; der Außenschaft 13 und mit ihm das Gelenk 16, die durch das Gelenk 16 definierte Schwenkachse und das Werkzeug 14 um die Längsachse 18 des Außenschafts 13 rotiert werden.

Am proximalen Ende 11 weist das medizinische Instrument 10 eine Handhabungseinrichtung 20 mit einem feststehenden Griffteil 21, einem ersten bewegbaren Griffteil 22 und einem zweiten bewegbaren Griffteil 23 auf. In Figur 1 deuten Pfeile an, dass das erste bewegbare Griffteil 22 und das zweite bewegbare Griffteil 23 jeweils um eine zugeordnete Schwenkachse senkrecht zur Zeichenebene der Figur 1 schwenkbar sind. Ferner weist die Handhabungseinrichtung 20 ein erstes Drehrad 24 und ein zweites Drehrad 25 auf, die (durch Pfeile angedeutet) jeweils um die Längsachse 18 des Außenschafts 13 rotierbar sind. Das erste Drehrad 24 ist an einer proximalen Seite der Handhabungseinrichtung 20 angeordnet, das zweite Drehrad 25 an einer distalen Seite. Die Anordnung und Ausgestaltung des feststehenden Griffteils 21, der bewegbaren Griffteile 22, 23 und der Drehräder 24, 25 kann im Sinne einer verbesserten Ergonomie von der Darstellung in Figur 1 deutlich abweichen.

Die bewegbaren Griffteile 22, 23 und die Drehräder 24, 25 sind derart mit dem Außenschaft 13, dem Werkzeug 14 bzw. dessen Maulteilen und dem Gelenk 16 gekoppelt, dass die oben genannten Freiheitsgrade insbesondere unabhängig voneinander und insbesondere gleichzeitig oder alternativ gesteuert werden können. Beispielsweise ist das zweite Drehrad 25 mit dem Außenschaft gekoppelt (insbesondere starr, lösbar oder unlösbar mit dem Außenschaft 13 verbunden), um eine Rotation des Außenschafts 13 um seine Längsachse 18 zu ermöglichen.

Das zweite bewegbare Griffteil 23 ist mittels einer in Figur 1 nicht dargestellten Kopplungseinrichtung mit einer ersten Halteeinrichtung 40 und mit einer zweiten Halteeinrichtung 50 gekoppelt. Das zweite bewegbare Griffteil 23 ist über die erste Halteeinrichtung 40 mit dem Außenschaft 13 und über die zweite Halteeinrichtung 50 mit einem Innenschaft gekoppelt. Der Außenschaft 13 und der Innenschaft sind derart mit dem Gelenk 16 nahe dem distalen Ende des Außenschafts 13 gekoppelt, dass eine mittels des zweiten bewegbaren Griffteils 23 hervorgerufene Relativbewegung des Außenschafts 13 und des Innenschafts parallel zur Längsachse 18 eine Abwinklung am Gelenk 16 steuert.

Das erste bewegbare Griffteil 22 und das erste Drehrad 24 sind mittels einer dritten Halteeinrichtung 60 mit einer Übertragungsstange und mittels der Übertragungsstange mit dem Werkzeug 14 und insbesondere dessen schwenkbaren Maulteilen gekoppelt. Durch eine Schwenkbewegung des ersten bewegbaren Griffteils 22 sind eine translatorische Bewegung der Übertragungsstange parallel zur Längsachse 18 und ein Öffnen oder Schließen der Maulteile des Werkzeugs 14 steuerbar. Durch Rotation des ersten Drehrads 24 sind eine Rotation der Übertragungsstange um die Längsachse 18 und eine Rotation des Werkzeugs 14 um seine Längsachse 15 steuerbar.

Alternativ ist eine andere Zuordnung der bewegbaren Griffteile 22, 23 und der Drehräder 24, 25 zu den Freiheitsgraden möglich. Zur Steuerung des Abknickens des distalen Endes des Außenschafts 13 am Gelenk 16 kann beispielsweise das zweite bewegbare Griffteil 23 durch ein weiteres Drehrad ersetzt werden. Ferner können mehrere Freiheitsgrade durch eine Betätigungseinrichtung gesteuert werden. Beispielsweise kann mittels eines axial verschiebbaren Drehrads abhängig von dessen axialer Position alternativ entweder das Abknicken des distalen Endes des Außenschafts 13 am Gelenk 16 oder die Rotation des Werkzeugs 14 um seine Längsachse 15 gesteuert werden.

Das medizinische Instrument 10 ist zerlegbar. Insbesondere kann der Außenschaft 13 zusammen mit dem Innenschaft und der Übertragungsstange von der Handhabungseinrichtung 20 getrennt werden. Die Halteeinrichtungen 40, 50, 60 sind ausgebildet, um die proximalen Enden des Außenschafts 13, des Innenschafts und der Übertragungsstange lösbar zu halten bzw. mit diesen lösbar mechanisch verbunden zu sein. Ferner können das Werkzeug 14 zusammen mit der Übertragungsstange und das Gelenk 16 zusammen mit dem Innenschaft vom Außenschaft 13 trennbar bzw. nach distal aus dem Außenschaft 13 entnehmbar sein.

Eine Betätigungseinrichtung 70 ist mit der Halteeinrichtungen 40, 50, 60 wirksam gekoppelt. Durch Betätigung der Betätigungseinrichtung 70, insbesondere durch ein manuelles Bewegen der Betätigungseinrichtung 70 parallel zur Längsachse 18 nach proximal, sind alle Verbindungen zwischen den Halteeinrichtungen 40, 50, 60 und dem jeweils zugeordneten proximalen Ende des Außenschafts 13, des Innenschafts bzw. der Übertragungsstange lösbar. Bei oder nach Betätigung der Betätigungseinrichtung 70 kann das proximale Ende des Außenschafts 13 zusammen mit dem proximalen Ende des Innenschafts und dem proximalen Ende der Übertragungsstange nach distal aus der Handhabungseinrichtung 20 herausgezogen werden oder durch Federkraft nach distal aus der Handhabungseinrichtung 20 ausgestoßen werden.

Die schwenkbaren Griffteile 22, 23 und die Drehräder 24, 25 sind derart mit den Halteeinrichtungen 40, 50, 60 mechanisch wirksam gekoppelt, dass die Halteeinrichtungen 40, 50, 60 zusammen mit dem jeweils zugeordneten proximalen Ende des Außenschafts 13 bzw. des Innenschafts bzw. der Übertragungsstange parallel zur Längsachse 18 verschoben und/oder um die Längsachse 18 rotiert werden können. Die Betätigungseinrichtung 70 ist mit den Halteeinrichtungen 40, 50, 60 derart mechanisch wirksam gekoppelt, dass eine manuelle Betätigung der Betätigungseinrichtung 70 ein Lösen der mechanischen Verbindungen zwischen den Halteeinrichtungen 40, 50, 60 und dem jeweils zugeordneten proximalen Ende des Außenschafts 13 bzw. des Innenschafts bzw. der Übertragungsstange bewirkt. Die mechanische Kopplung zwischen der Betätigungseinrichtung 70 einerseits und den Halteeinrichtungen 40, 50, 60 andererseits ist unten mit Bezug auf die Figuren 2 bis 7 beschrieben, und zwar unabhängig von der mechanischen Kopplung der schwenkbaren Griffteile 22, 23 und der Drehräder 24, 25 mit den Halteeinrichtungen 40, 50, 60.

Figur 2 zeigt eine schematische Schnittdarstellung eines Teils einer Handhabungseinrichtung, insbesondere der oben anhand der Figur 1 dargestellten Handhabungseinrichtung 20. Die in Figur 2 dargestellte Schnittebene enthält die Längsachse 18 des Außenschafts (vgl. Figur 1). Der in Figur 2 dargestellte Teil der Handhabungseinrichtung umfasst die bereits oben anhand der Figur 1 beschriebenen Halteeinrichtungen 40, 50, 60, die Betätigungseinrichtung 70 und die mechanische Kopplung zwischen diesen. Die mechanische Kopplung der Halteeinrichtungen 40, 50, 60 mit in Figur 2 nicht dargestellten bewegbaren Griffteilen und Drehrädern ist in Figur 2 nicht dargestellt. Die Halteeinrichtungen 40, 50, 60 können mit bewegbaren Griffteilen und Drehrädern gekoppelt sein, die sich hinsichtlich ihrer Anordnung und Ausgestaltung von den oben anhand der Figur 1 dargestellten unterscheiden.

In Figur 2 sind ein Teilbereich eines feststehenden Gehäuseteils 27 und eines rotierbaren Gehäuseteils 28 der Handhabungseinrichtung dargestellt. Das rotierbare Gehäuseteil 28 ist derart formschlüssig mit dem feststehenden Gehäuseteil 27 verbunden, dass es relativ zu diesem um die Längsachse 18 rotierbar ist. Im feststehenden Gehäuseteil 27 sind die erste Halteeinrichtung 40 und die zweite Halteeinrichtung 50 angeordnet, im rotierbaren Gehäuseteil 28 ist die dritte Halteeinrichtung 60 angeordnet. Von der distalen Seite (in den Figuren 1 und 2: links) aus ragen das proximale Ende 34 eines Außenschafts, das proximale Ende 35 eines Innenschafts und das proximale Ende 36 einer Übertragungsstange in die Handhabungseinrichtung. Die Übertragungsstange ist koaxial in dem im Wesentlichen rohrförmigen Innenschaft angeordnet. Die Übertragungsstange und der Innenschaft sind koaxial in dem im Wesentlichen rohrförmigen Außenschaft angeordnet. Die Längsachse 18 ist somit gleichzeitig Längsachse des proximalen Endes 34 des Außenschafts, des proximalen Endes 35 des Innenschafts und des proximalen Endes 36 der Übertragungsstange. Das proximale Ende 34 des Außenschafts, das proximale Ende 35 des Innenschafts und das proximale Ende 36 der Übertragungsstange sind insbesondere jeweils im Wesentlichen rotationssymmetrisch zur Längsachse 18. Distal der Handhabungseinrichtung können der Außenschaft, der Innenschaft und die Übertragungsstange gekrümmt sein, wobei der Innenschaft und die Übertragungsstange insbesondere flexibel ausgebildet sind.

Die Halteeinrichtungen 40, 50, 60 umfassen jeweils eine in Figur 2 nicht mit einem eigenen Bezugszeichen versehene Ausnehmung, deren Gestalt an die Gestalt des proximalen Endes 34 des Außenschafts bzw. an die Gestalt des proximalen Endes 35 des Innenschafts bzw. an das proximale Ende 36 der Übertragungsstange angepasst ist. Diese Ausnehmungen erstrecken sich im Wesentlichen parallel zur Längsachse 18 und können nach distal trichterförmig erweitert sein, wie dies für die zweite Halteeinrichtung 50 und die dritte Halteeinrichtung 60 in Figur 2 angedeutet ist. Die Ausnehmungen in den Halteeinrichtungen 40, 50, 60 sind insbesondere durch stufenförmige Absätze oder Stirnwände so ausgebildet, dass sie für das proximale Ende 34 des Außenschafts bzw. für das proximale Ende 35 des Innenschafts bzw. für das proximale Ende 36 der Übertragungsstange jeweils einen Anschlag nach proximal bilden. Dies ermöglicht eine Übertragung einer nach distal gerichteten Kraft (Schubkraft) von einer Halteeinrichtung 40, 50, 60 auf das zugeordnete proximale Ende 34, 35, 36 des Außenschafts, des Innenschafts bzw. der Übertragungsstange.

Die Querschnitte des proximalen Endes 34 des Außenschafts, des proximalen Endes 35 des Innenschafts und des proximalen Endes 36 der Übertragungsstange und die korrespondierenden Querschnitte der Ausnehmungen in den zugeordneten Halteeinrichtungen 40, 50, 60 in Ebenen senkrecht zur Längsachse 18 können jeweils kreisförmig sein oder eine zur Längsachse 18 nicht-rotationssymmetrische Gestalt aufweisen. Im Fall von korrespondierenden nicht-rotationssymmetrischen Querschnitten kann ein Drehmoment übertragen werden.

Insbesondere sind das proximale Ende 34 des Außenschafts und die korrespondierende Ausnehmung in der ersten Halteeinrichtung 40 sowie das proximale Ende 35 des Innenschafts und die korrespondierende Ausnehmung in der zweiten Halteeinrichtung 50 jeweils rotationssymmetrisch zur Längsachse 18 ausgebildet. Deshalb können das proximale Ende 34 des Außenschafts relativ zur ersten Halteeinrichtung 40 frei rotiert werden und das proximale Ende 35 des Innenschafts relativ zur zweiten Halteeinrichtung 50 frei um die Längsachse 18 rotiert werden. Das proximale Ende 36 der Übertragungsstange und die korrespondierende Ausnehmung der zugeordneten dritten Halteeinrichtung 60 sind insbesondere nicht rotationssymmetrisch zur Längsachse 18 ausgebildet, um eine Übertragung eines Drehmoments zwischen der dritten Halteeinrichtung 60 und dem proximalen Ende 36 der Übertragungsstange zu ermöglichen.

In der ersten Halteeinrichtung 40 ist ein Riegel 41 angeordnet, der in einer Bewegungsrichtung 47 senkrecht zur Längsachse 18 verschiebbar ist und durch eine Feder 42 in die in Figur 2 dargestellte Position geschoben wird, in der der Riegel 41 in eine Nut am proximalen Ende 34 des Außenschafts eingreift. Ein von der Feder 42 abgewandtes Ende des Riegels 41 greift durch einen Schlitz 45 im feststehenden Gehäuseteil 27 hindurch. In der zweiten Halteeinrichtung 50 ist ein Riegel 51 angeordnet, der in einer Bewegungsrichtung 57 senkrecht zur Längsachse 18 verschiebbar ist und durch eine Feder 52 in die in Figur 2 dargestellte Position geschoben wird, in der der Riegel 51 in eine Nut am proximalen Ende 35 des Innenschafts eingreift. Ein von der Feder 52 abgewandtes Ende des Riegels 51 greift durch einen Schlitz 55 im feststehenden Gehäuseteil 27 hindurch. In der dritten Halteeinrichtung 60 ist ein Riegel 61 angeordnet, der in einer Bewegungsrichtung 67 senkrecht zur Längsachse 18 verschiebbar ist und durch eine Feder 62 in die in Figur 2 dargestellte Position geschoben wird, in der der Riegel 61 in eine Nut am proximalen Ende 36 der Übertragungsstange eingreift. Ein von der Feder 62 abgewandtes Ende des Riegels 61 greift durch einen Schlitz 65 hindurch. Jeder Riegel 41, 51, 61 ist insbesondere ausgebildet als Platte mit einem Loch bzw. einer Durchgangsbohrung, durch die das proximale Ende 34 des Außenschafts bzw. das proximale Ende 35 des Innenschafts bzw. das proximale Ende 36 der Übertragungsstange hindurchgreift.

Die Betätigungseinrichtung 70 umgibt das rotierbare Gehäuseteil 28 im Wesentlichen becherförmig. An der Außenseite der Betätigungseinrichtung 70 kann eine in Figur 2 nicht dargestellte Riffelung vorgesehen sein, um ein manuelles Greifen und Ziehen der Betätigungseinrichtung 70 nach proximal zu vereinfachen. An der Innenseite weist die Betätigungseinrichtung 70 eine Gleitfläche 71 mit einem konischen Bereich auf. Das durch den Schlitz 65 im rotierbaren Gehäuseteil 27 hindurchgreifende Ende des Riegels 61 der dritten Halteeinrichtung 60 liegt an der Gleitfläche 71 an.

Die Betätigungseinrichtung 70 ist mittels einer Verbindungsstange 72 mit einem Schlitten 73 starr verbunden, der überwiegend innerhalb des feststehenden Gehäuseteils 27 angeordnet ist. Die Verbindungsstange 72 ist mit der Betätigungseinrichtung 70 und mit dem Schlitten 73 jeweils stoffschlüssig, formschlüssig und/oder reibschlüssig verbunden. Die Betätigungseinrichtung 70 und der Schlitten 73 sind gemeinsam in einer Bewegungsrichtung 77 parallel zur Längsachse 18 bewegbar bzw. verschiebbar. Eine in Figur 2 nicht dargestellte Feder oder ein anderes elastisches Element kann vorgesehen sein, um die Betätigungseinrichtung 70 und den Schlitten 73 in die in Figur 2 dargestellte Position zu schieben oder zu ziehen.

Zwischen dem Schlitten 73 einerseits und der ersten Halteeinrichtung 40 und der zweiten Halteeinrichtung 50 andererseits ist eine Hubplatte 81 angeordnet. Eine Gleitfläche 75 am Schlitten 73 liegt an einer Gleitfläche 85 an der Hubplatte 81 an. Die Gleitfläche 75 am Schlitten 73 und die Gleitfläche 85 an der Hubplatte 81 weisen jeweils einen Bereich auf, der nicht parallel zur Bewegungsrichtung 77 bzw. relativ zur Bewegungsrichtung 77 des Schlittens 73 geneigt ist. Die von den Federn 42, 52 abgewandten Enden der Riegel 41, 51 der ersten Halteeinrichtung 40 und der zweiten Halteeinrichtung 50 liegen an einer von der Gleitfläche 85 abgewandten Anlagefläche 86 an der Hubplatte 81 an. Die Hubplatte 81 ist in einer Bewegungsrichtung 87 senkrecht zur Längsachse 18 und zur Bewegungsrichtung 77 des Schlittens 73 sowie parallel zu den Bewegungsrichtungen 47, 57 der Riegel 41, 51 der ersten Halteeinrichtung 40 und der zweiten Halteeinrichtung 50 bewegbar im feststehenden Gehäuseteil 27 geführt.

Figur 3 zeigt eine weitere schematische Darstellung des in Figur 2 gezeigten Teils einer Handhabungseinrichtung, die insbesondere hinsichtlich der Schnittebene der Figur 2 entspricht. Bei dem in den Figuren 2 und 3 dargestellten Beispiel sind die erste Halteeinrichtung 40 zusammen mit dem proximalen Ende 34 des Außenschafts, die zweite Halteeinrichtung 50 zusammen mit dem proximalen Ende 35 des Innenschafts und die dritte Halteeinrichtung 60 zusammen mit dem proximalen Ende 36 der Übertragungsstange jeweils parallel zur Längsachse 18 verschiebbar. Ferner ist bei dem in den Figuren 2 und 3 dargestellten Beispiel die dritte Halteeinrichtung 60 zusammen mit der Übertragungsstange um die Längsachse 18 rotierbar. Die Darstellung in Figur 3 unterscheidet sich von der Darstellung in Figur 2 dadurch, dass die erste Halteeinrichtung 40 zusammen mit dem proximalen Ende 34 des Außenschafts von der in Figur 2 dargestellten distalen Position in eine proximale Position; die zweite Halteeinrichtung 50 zusammen mit dem proximalen Ende 35 des Innenschafts von der in Figur 2 dargestellten proximalen Position in eine distale Position; und die dritte Halteeinrichtung 60 zusammen mit dem proximalen Ende 36 der Übertragungsstange von der in Figur 2 dargestellten distalen Position in eine proximale Position verschoben sind. Außerdem ist die dritte Halteeinrichtung 60 zusammen mit dem rotierbaren Gehäuseteil 28 und der Übertragungsstange relativ zu der in Figur 2 dargestellten Position um 180 Grad um die Längsachse 18 rotiert. Es ist erkennbar, dass die von den Federn 42, 52, 62 abgewandten Enden der Riegel 41, 51, 61 auch bei den in den Figuren 3 dargestellten Positionen der Halteeinrichtungen 40, 50, 60 an der Anlagefläche 85 an der Hubplatte 81 bzw. an der Gleitfläche 71 an der Betätigungseinrichtung 70 anliegen.

Wie bereits erwähnt, ist in den Figuren 1 bis 3 die wirksame mechanische Kopplung zwischen den Halteeinrichtungen 40, 50, 60 einerseits und den bewegbaren Griffteilen 22, 23 und den Drehrädern 24, 25 andererseits nicht dargestellt. Lediglich beispielhaft ist in den Figuren 2 und 3 eine Welle 69 angedeutet, deren distales Ende mit der dritten Halteeinrichtung 60 starr verbunden ist. Bei dem in Figur 1 dargestellten Beispiel kann das in den Figuren 2 und 3 nicht dargestellte proximale Ende mit dem ersten bewegbaren Griffteil 22 und dem ersten Drehrad 24 gekoppelt sein. Die erste Halteeinrichtung 40 und die zweite Halteeinrichtung 50 können unabhängig voneinander bewegbar oder - beispielsweise über gegenläufige Gewinde - so miteinander gekoppelt sein, dass sie stets gegenläufig bewegt werden.

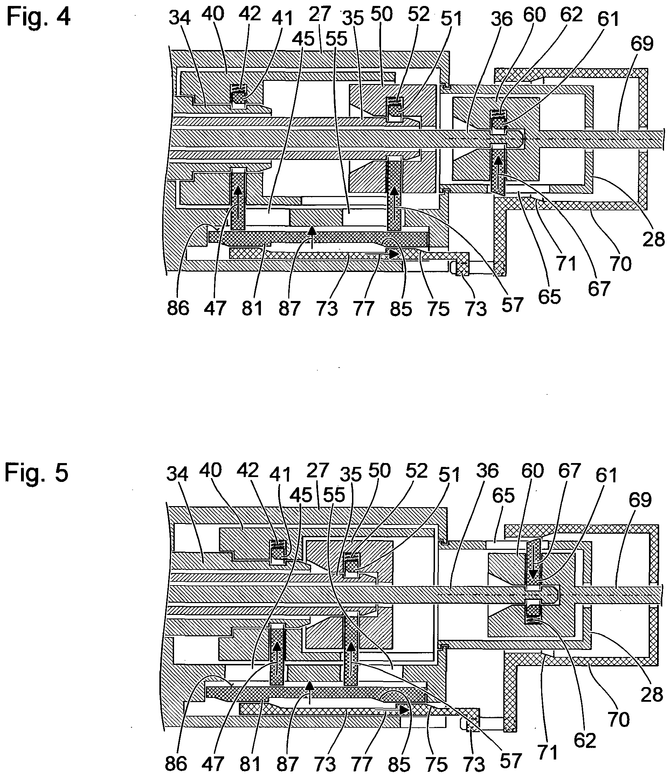

Die Figuren 4 und 5 zeigen je eine weitere schematische Schnittdarstellung des Beispiels aus den Figuren 2 und 3. Die Darstellungen in den Figuren 4 und 5 entsprechen insbesondere hinsichtlich der dargestellten Schnittebenen den Figuren 2 und 3. Die erste Halteeinrichtung 40 und das proximale Ende 34 des Außenschafts nehmen in der Darstellung in Figur 4 die auch in Figur 2 gezeigten distalen Positionen und in der Darstellung in Figur 5 die auch in Figur 3 gezeigten proximalen Positionen ein. Die zweite Halteeinrichtung 50 und das proximale Ende 35 des Innenschafts nehmen in der Darstellung in Figur 4 die in Figur 2 gezeigten proximalen Positionen und in Figur 5 die in Figur 3 gezeigten distalen Positionen ein. Die dritte Halteeinrichtung 60 und das proximale Ende 36 der Übertragungsstange nehmen in der Darstellung in Figur 4 die in Figur 2 gezeigte Positionen und in der Darstellung in Figur 5 die in Figur 3 gezeigten Positionen ein. Insoweit entsprechen die Figur 4 der Figur 2 und die Figur 5 der Figur 3.

Die Darstellungen in den Figuren 4 und 5 unterscheiden sich von den Darstellungen in den Figuren 2 und 3 dadurch, dass die Betätigungseinrichtung 70 betätigt, nämlich nach proximal verschoben ist. In den Figuren 4 und 5 ist erkennbar, dass diese Verschiebung vermittels der Verbindungsstange 72 eine entsprechende Verschiebung des Schlittens 73 und vermittels der Keilwirkung der geneigten Abschnitte der Gleitflächen 75, 85 eine Verschiebung der Hubplatte 81 in der Bewegungsrichtung 87 zu der ersten Halteeinrichtung 40 und der zweiten Halteeinrichtung 50 hin bewirkt. Diese Verschiebung der Hubplatte 81 bewirkt eine entsprechende Verschiebung der Riegel 41, 51 gegen die Kräfte der Federn 42, 52, da die von den Federn 42, 52 abgewandten Enden der Riegel 41, 51 an der Anlagefläche 86 der Hubplatte 81 anliegen. Die Keilwirkung des konischen Bereichs der Gleitfläche 71 an der Innenseite der Betätigungseinrichtung 70 bewirkt eine entsprechende Verschiebung des Riegels 61 der dritten Halteeinrichtung 60 gegen die Kraft der Feder 62. Die Verschiebung der Betätigungseinrichtung 70 nach proximal zu den in den Figuren 4 und 5 gezeigten Positionen hin, bewirkt somit eine Verschiebung der Riegel 41, 51, 61 der Halteeinrichtungen 40, 50, 60 zu den in Figuren 4 und 5 gezeigten Positionen hin.

In den in den Figuren 4 und 5 gezeigten Positionen greifen die Riegel 41, 51, 61 nicht mehr in die Nuten am proximalen Ende 34 des Außenschafts bzw. am proximalen Ende 35 des Innenschafts bzw. am proximalen Ende 36 der Übertragungsstange ein. Damit sind die mechanischen Verbindungen gelöst bzw. entriegelt, und das proximale Ende 34 des Außenschafts, das proximale Ende 35 des Innenschafts und das proximale Ende 36 der Übertragungsstange sind freigegeben und können nach distal aus der Handhabungseinrichtung herausgezogen werden. Im Vergleich der Figuren 4 und 5 ist erkennbar, dass durch die dargestellte Verschiebung der Betätigungseinrichtung 70 nach proximal die mechanische Verbindung der ersten Halteeinrichtung 40 mit dem proximalen Ende 34 des Außenschafts, die mechanische Verbindung der zweiten Halteeinrichtung 50 mit dem proximalen Ende 35 des Innenschafts und die mechanische Verbindung der dritten Halteeinrichtung 60 mit dem proximalen Ende 36 der Übertragungsstange im Wesentlichen gleichzeitig und unabhängig von den Positionen der Halteeinrichtungen 40, 50, 60 gelöst werden.

In der Zusammenschau der Figuren 2 bis 5 ist erkennbar, dass die Gleitfläche 71 an der Betätigungseinrichtung 70, die Verbindungsstange 72, der Schlitten 73 und die Hubplatte 81 eine Kopplungseinrichtung zur mechanischen Kopplung der Betätigungseinrichtung 70 mit den Riegeln 41, 51, 61 der Halteeinrichtungen 40, 50, 60 bilden.

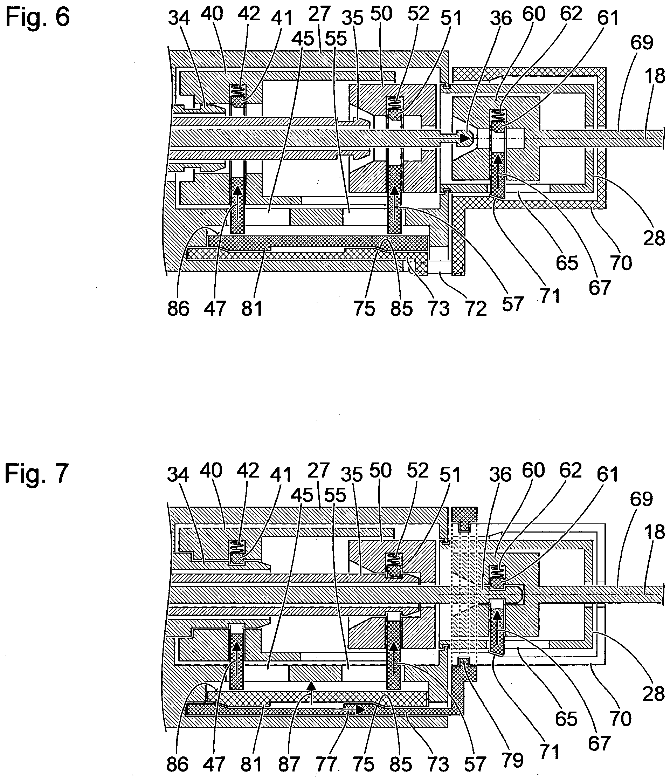

Figur 6 zeigt eine weitere schematische Schnittdarstellung des auch in den Figuren 2 bis 5 gezeigten Teils einer Handhabungseinrichtung. Die Darstellung in Figur 6 entspricht insbesondere hinsichtlich der Schnittebene den Darstellungen in den Figuren 2 bis 5. Die Halteeinrichtungen 40, 50, 60, die Betätigungseinrichtung 70, der Schlitten 73 und die Hubplatte 81 nehmen die bereits in Figur 2 gezeigten Positionen ein. Die Darstellung in Figur 6 unterscheidet sich von der Darstellung in Figur 2 dadurch, dass das proximale Ende 34 des Außenschafts, das proximale Ende 35 des Innenschafts und das proximale Ende 36 der Übertragungsstange nicht von den Halteeinrichtungen 40, 50, 60 gehalten sind. Das proximale Ende 34 des Außenschafts, das proximale Ende 35 des Innenschafts und das proximale Ende 36 der Übertragungsstange sind stattdessen in nach distal verschobenen Positionen gezeigt.

Bei dem in Figur 6 dargestellten Zustand nehmen die Riegel 41, 51, 61 aufgrund der Wirkung der Federn 42, 52, 62 Positionen ein, die den in den Figuren 2 und 3 gezeigten Halte- bzw. Verriegelungspositionen entsprechen.

Durch Bewegung des proximalen Endes 34 des Außenschafts, des proximalen Endes 35 des Innenschafts und des proximalen Endes 36 der Übertragungsstange nach proximal können diese von den Halteeinrichtungen 40, 50, 60 aufgenommen und in den Halteeinrichtungen 40, 50, 60 verriegelt werden. Dazu ist keine Betätigung der Betätigungseinrichtung 70 erforderlich. Gegenüber der Längsachse 18 geneigte Gleitflächen am proximalen Ende 34 des Außenschafts, am proximalen Ende 35 des Innenschafts, am proximalen Ende 36 der Übertragungsstange und an den Riegeln 41, 51, 61 der Halteeinrichtungen 40, 50, 60 bewirken eine vorübergehende Verschiebung der Riegel 41, 51, 61 in den Bewegungsrichtungen 47, 57, 67 gegen die Kräfte der Federn 42, 52, 62. Die Riegel 41, 51, 61 nehmen ohne Betätigung der Betätigungseinrichtung 70 vorübergehend ihre in den Figuren 3 und 5 gezeigten Lösepositionen ein bis die Riegel 41, 51, 61 wie in Figur 2 gezeigt, in die Nuten am proximalen Ende 34 des Außenschafts, am proximalen Ende 35 des Innenschafts und am proximalen Ende 36 der Übertragungsstange eingreifen und diese formschlüssig halten. Dabei werden die von den Federn 42, 52 abgewandten Enden der Riegel 41, 51 der ersten Halteeinrichtung 40 und der zweiten Halteeinrichtung 50 vorübergehend von der Anlagefläche 86 an der Hubplatte 81 abgehoben.

Figur 7 zeigt eine schematische Schnittdarstellung eines Teils eines weiteren Ausführungsbeispiels einer Halteeinrichtung, das in einigen Merkmalen dem Ausführungsbeispiel der Figuren 2 bis 6 ähnelt. Die Darstellung in Figur 7 entspricht insbesondere hinsichtlich der Schnittebene den Darstellungen der Figuren 2 bis 6.

Das in Figur 7 dargestellte Ausführungsbeispiel ähnelt dem Ausführungsbeispiel der Figuren 2 bis 6 insbesondere hinsichtlich der Ausgestaltung der Halteeinrichtungen 40, 50, 60, des Schlittens 73 und der Hubplatte 81. Das Ausführungsbeispiel der Figur 3 unterscheidet sich von dem Ausführungsbeispiel der Figuren 2 bis 6 insbesondere dadurch, dass die Betätigungseinrichtung 70 relativ zum Schlitten 73 um die Längsachse 18 rotierbar ist. Dazu ist ein Drehlager 79 zwischen der Betätigungseinrichtung 70 und dem Schlitten 73 vorgesehen, das beide formschlüssig und relativ zueinander um die Längsachse 18 rotierbar verbindet.

In Figur 7 ist an der Innenseite der Betätigungseinrichtung 70, ähnlich wie bei dem Ausführungsbeispiel der Figuren 2 bis 6 eine Gleitfläche 71 mit einem konischen Abschnitt gezeigt. Da die Betätigungseinrichtung 70 relativ zum Schlitten 73 um die Längsachse 18 rotierbar ist, kann die Betätigungseinrichtung 70 mit dem rotierbaren Gehäuseteil 28 und der dritten Halteeinrichtung 60 über in Figur 7 nicht gezeigte Merkmale so gekoppelt sein, dass eine Rotation der Betätigungseinrichtung 70 um die Längsachse 18 eine entsprechende Rotation der dritten Halteeinrichtung 60 und damit auch des in der dritten Halteeinrichtung 60 gehaltenen proximalen Endes 36 der Übertragungsstange um die Längsachse 18 bewirkt. In diesem Fall kann anstelle eines konischen Abschnitts abweichend von der Darstellung in Figur 7 lediglich ein streifenförmiger rampenförmiger Abschnitt an der Gleitfläche 71 an der Betätigungseinrichtung 70 vorgesehen sein.

### Bezugszeichen

- 10: medizinisches Instrument
- 11: proximales Ende des medizinischen Instruments 10
- 12: distales Ende des medizinischen Instruments 10
- 13: Außenschaft des medizinischen Instruments 10
- 14: Werkzeug
- 15: Längsachse des Werkzeugs 14
- 16: Gelenk am Außenschaft 13
- 18: Längsachse des Außenschafts 13
- 20: Handhabungseinrichtung des medizinischen Instruments 10
- 21: feststehendes Griffteil der Handhabungseinrichtung 20
- 22: erstes bewegbares Griffteil an der Handhabungseinrichtung 20
- 23: zweites bewegbares Griffteil an der Handhabungseinrichtung 20
- 24: erstes Drehrad an der Handhabungseinrichtung 20
- 25: zweites Drehrad an der Handhabungseinrichtung 20
- 27: feststehender Gehäuseteil der Handhabungseinrichtung 20
- 28: rotierbarer Gehäuseteil der Handhabungseinrichtung 20
- 34: proximales Ende des Außenschafts 13
- 35: Innenschaft, proximales Ende des Innenschafts
- 36: Übertragungsstange, proximales Ende der Übertragungsstange
- 40: erste Halteeinrichtung für proximales Ende des Außenschafts 34
- 41: Riegel der ersten Halteeinrichtung 40
- 42: Feder am Riegel 41
- 45: Schlitz im feststehenden Gehäuseteil 27 für Riegel 41
- 47: Bewegungsrichtung des Riegels 41
- 50: zweite Halteeinrichtung für proximales Ende des Innenschafts 35
- 51: Riegel der zweiten Halteeinrichtung 50
- 52: Feder am Riegel 51
- 55: Schlitz im feststehenden Gehäuseteil 27 für Riegel 51
- 57: Bewegungsrichtung des Riegels 51
- 60: dritte Halteeinrichtung für proximales Ende der Übertragungsstange 36
- 61: Riegel der dritten Halteeinrichtung 60
- 62: Feder am Riegel 61
- 65: Schlitz im rotierbaren Gehäuseteil 27 für Riegel 61
- 67: Bewegungsrichtung des Riegels 61
- 69: Welle an der dritten Halteeinrichtung 60
- 70: Betätigungseinrichtung für Entriegelung
- 71: Gleitfläche an der Betätigungseinrichtung 70
- 72: Verbindungsstange
- 73: Schlitten
- 75: Gleitfläche am Schlitten 73
- 77: Bewegungsrichtung des Schlitten 73
- 79: Drehlager für rotierbaren Teil 78
- 81: Hubplatte
- 85: Gleitfläche an der Hubplatte 81
- 86: Anlagefläche der Hubplatte 81
- 87: Bewegungsrichtung der Hubplatte 81

## Patentansprüche

1. **Handhabungseinrichtung** (20) zur mechanischen Verbindung mit dem proximalen Ende (34) eines Schafts (13), zur Bildung eines medizinischen Instruments (10), mit:
einer **Halteeinrichtung** (40, 50, 60) zum lösbaren Halten des proximalen Endes (34) eines Schafts (13) oder des proximalen Endes (35, 36) einer Übertragungseinrichtung zum Übertragen zumindest entweder einer Kraft oder eines Drehmoments;
einer manuell betätigbaren **Betätigungseinrichtung** (70);
einer **Kopplungseinrichtung** (71, 72, 73, 75, 81, 85) zur mechanischen Kopplung der Betätigungseinrichtung (70) mit der Halteeinrichtung (40, 50, 60) derart, dass eine manuelle Betätigung der Betätigungseinrichtung (70) ein Lösen der Halteeinrichtung (40, 50, 60) bewirkt,
wobei die **Halteeinrichtung** (40, 50, 60) zusammen mit einem von der Halteeinrichtung (40, 50, 60) gehaltenen proximalen Ende (34, 35, 36) eines Schafts (13) oder einer Übertragungseinrichtung relativ zu der Betätigungseinrichtung (29) parallel zur Längsachse (18) des Schafts (13) bzw. der Übertragungseinrichtung **verschiebbar** ist,
wobei die Kopplungseinrichtung (71, 72, 73, 75, 81, 85) ausgebildet ist, um die Betätigungseinrichtung (70) **unabhängig von der Position** der verschiebbaren Halteeinrichtung (40, 50, 60) relativ zur Betätigungseinrichtung (70) mit der bewegbaren Halteeinrichtung (40, 50, 60) derart zu koppeln, dass die Halteeinrichtung lösbar ist, **dadurch gekennzeichnet, dass** eine **Mehrzahl** von **Halteeinrichtungen** (40, 50, 60) vorgesehen ist, wobei jede einzelne der Mehrzahl von Halteeinrichtungen (40, 50, 60) zum lösbaren Halten des proximalen Endes (34) eines Schafts (13) oder des proximalen Endes (35, 36) einer Übertragungseinrichtung zum Übertragen zumindest entweder einer Kraft oder eines Drehmoments in dem Schaft (13) ausgebildet ist;
die **Kopplungseinrichtung** (71, 72, 73, 75, 81, 85) ausgebildet ist zur mechanischen Kopplung der Betätigungseinrichtung (70) mit der Mehrzahl von Halteeinrichtungen (40, 50, 60) derart, dass eine manuelle Betätigung der Betätigungseinrichtung (70) ein Lösen der Mehrzahl von Halteeinrichtungen (40, 50, 60) bewirkt.

2. Handhabungseinrichtung (20) nach dem vorangehenden Anspruch, bei der die bewegbare Halteeinrichtung (40, 50, 60) zusammen mit einem von der bewegbaren Halteeinrichtung (40, 50, 60) gehaltenen proximalen Ende (34, 35, 36) eines Schafts (13) oder einer Übertragungseinrichtung relativ zu der Betätigungseinrichtung (70) um eine Längsachse (18) des Schafts (13) bzw. der Übertragungseinrichtung **rotierbar** ist.

3. Handhabungseinrichtung (20) nach dem vorangehenden Anspruch, bei der eine **erste Halteeinrichtung** (40) der Mehrzahl von Halteeinrichtungen (40, 50, 60) ausgebildet ist, um ein proximales Ende (34) eines **Schafts** (13) zu halten, eine **zweite Halteeinrichtung** (50, 60) der Mehrzahl von Halteeinrichtungen (40, 50, 60) ausgebildet ist, um ein proximales Ende (35, 36) einer **Übertragungseinrichtung** zum Übertragen zumindest entweder einer Kraft oder eines Drehmoments zwischen der Handhabungseinrichtung (20) und einem Werkzeug (14) am distalen Ende des Schafts (13) zu halten.

4. Handhabungseinrichtung (20) nach einem der vorangehenden Ansprüche, bei der die **Betätigungseinrichtung** (70) zur Betätigung in Richtung **parallel zur Längsachse** (18) eines mit der Handhabungseinrichtung (20) verbundenen Schafts (13) manuell verschiebbar ist.

5. Handhabungseinrichtung (20) nach einem der vorangehenden Ansprüche, bei der die Kopplungseinrichtung aufweist:
ein **Kopplungselement** (70, 72, 73), das in einer ersten Bewegungsrichtung (77) verschiebbar ist,
eine **Gleitfläche** (71, 75) mit einem Bereich, der nicht parallel zur ersten Bewegungsrichtung (77) ist, um eine Verschiebung des Kopplungselements (70, 73) in der ersten Bewegungsrichtung (77) in eine Bewegung eines Riegels (41, 51, 61) in einer zweiten Bewegungsrichtung (87), die von der ersten Bewegungsrichtung (77) verschieden ist, umzusetzen.

6. Handhabungseinrichtung (20) nach dem vorangehenden Anspruch, bei der der Riegel (61) **an der Gleitfläche** (71) **anliegt.**

7. Handhabungseinrichtung (20) nach Anspruch 5, ferner mit:
einem **weiteren Kopplungselement** (81), das an dem Kopplungselement (73) anliegt und in der zweiten Bewegungsrichtung (87) bewegbar ist, wobei der Riegel (41, 51, 61) mit dem weiteren Kopplungselement (81) mechanisch gekoppelt ist.

8. Handhabungseinrichtung (20) nach dem vorangehenden Anspruch, bei der entweder das weitere Kopplungselement (81) die **Gleitfläche** aufweist oder die Gleitfläche (75) an dem weiteren Kopplungselement (81) gleitend anliegt.

9. Handhabungseinrichtung (20) nach Anspruch 7 oder 8 in Rückbezug auf einen der Ansprüche 3 bis 6, bei der das weitere Kopplungselement (81) eine **Anlagefläche** (86) aufweist, die parallel zur Längsachse (18) eines von der bewegbaren Halteeinrichtung (40, 50) gehaltenen proximalen Endes (34, 35) eines Schafts (13) oder einer Übertragungseinrichtung ist, und an der ein Riegel (41, 51, 61) der bewegbaren Halteeinrichtung (40, 50) anliegt.

10. Handhabungseinrichtung (20) nach einem der Ansprüche 5 bis 9, bei der die zweite Bewegungsrichtung (87) zumindest entweder **senkrecht** zur ersten Bewegungsrichtung (77) oder senkrecht zur Längsachse (18) eines mit der Handhabungseinrichtung (20) verbundenen Schafts (13) ist.

11. **Medizinisches Instrument** (10) mit einer Handhabungseinrichtung (20) nach einem der vorangehenden Ansprüche.

## Claims

1. Manipulation device (20) for mechanical connection to the proximal end (34) of a shaft (13), for forming a medical instrument (10), with:
a retaining device (40, 50, 60) for releasably retaining the proximal end (34) of a shaft (13) or the proximal end (35, 36) of a transmission device for transmitting at least either a force or a torque;
a manually activatable actuation device (70);
a coupling device (71, 72, 73, 75, 81, 85) for mechanically coupling the actuation device (70) to the retaining device (40, 50, 60) in such a way that a manual activation of the actuation device (70) causes a release of the retaining device (40, 50, 60),
wherein the retaining device (40, 50, 60), together with a proximal end (34, 35, 36) of a shaft (13) or of a transmission device retained by the retaining device (40, 50, 60), is displaceable relative to the actuation device (29) parallel to the longitudinal axis (18) of the shaft (13) or of the transmission device,
wherein the coupling device (71, 72, 73, 75, 81, 85) is designed to couple the actuation device (70) to the movable retaining device (40, 50, 60), independently of the position of the displaceable retaining device (40, 50, 60) relative to the actuation device (70), in such a way that the retaining device is releasable,
**characterized in that** a plurality of retaining devices (40, 50, 60) are provided, wherein each one of the plurality of retaining devices (40, 50, 60) for releasably retaining the proximal end (34) of a shaft (13) or the proximal end (35, 36) of a transmission device, is designed to transmit at least either a force or a torque in the shaft (13) ;
the coupling device (71, 72, 73, 75, 81, 85) is designed to mechanically couple the actuation device (70) to the plurality of retaining devices (40, 50, 60) in such a way that a manual activation of the actuation device (70) causes a release of the plurality of retaining devices (40, 50, 60).

2. Manipulation device (20) according to the preceding claim, in which the movable retaining device (40, 50, 60), together with a proximal end (34, 35, 36) of a shaft (13) or of a transmission device retained by the movable retaining device, (40, 50, 60), is rotatable relative to the actuation device (70) about a longitudinal axis (18) of the shaft (13) or of the transmission device.

3. Manipulation device (20) according to the preceding claim, in which
a first retaining device (40) of the plurality of retaining devices (40, 50, 60) is designed to retain a proximal end (34) of a shaft (13),
a second retaining device (50, 60) of the plurality of retaining devices (40, 50, 60) is designed to retain a proximal end (35, 36) of a transmission device for transmitting at least either a force or a torque between the manipulation device (20) and a tool (14) at the distal end of the shaft (13).

4. Manipulation device (20) according to one of the preceding claims, in which the actuation device (70) for the activation is manually displaceable in a direction parallel to the longitudinal axis (18) of a shaft (13) connected to the manipulation device (20).

5. Manipulation device (20) according to one of the preceding claims, in which the coupling device has:
a coupling element (70, 72, 73), which is displaceable in a first direction of movement (77),
a slide surface (71, 75) with a region that is not parallel to the first direction of movement (77), in order to convert a displacement of the coupling element (70, 73) in the first direction of movement (77) into a movement of a bolt (41, 51, 61) in a second direction of movement (87), which is different than the first direction of movement (77).

6. Manipulation device (20) according to the preceding claim, in which the bolt (61) bears on the slide surface (71).

7. Manipulation device (20) according to Claim 5, also with:
a further coupling element (81), which bears on the coupling element (73) and is movable in the second direction of movement (87), wherein the bolt (41, 51, 61) is mechanically coupled to the further coupling element (81).

8. Manipulation device (20) according to the preceding claim, in which either the further coupling element (81) has the slide surface, or the slide surface (75) bears slidingly on the further coupling element (81).

9. Manipulation device (20) according to Claim 7 or 8 with back-reference to one of Claims 3 to 6, in which the further coupling element (81) has a support surface (86), which is parallel to the longitudinal axis (18) of a proximal end (34, 35) of a shaft (13) or a transmission device retained by the movable retaining device (40, 50), and on which a bolt (41, 51, 61) of the movable retaining device (40, 50) bears.

10. Manipulation device (20) according to one of Claims 5 to 9, in which the second direction of movement (87) is at least either perpendicular to the first direction of movement (77) or perpendicular to the longitudinal axis (18) of a shaft (13) connected to the manipulation device (20).

11. Medical instrument (10) with a manipulation device (20) according to one of the preceding claims.

## Revendications

1. Dispositif de manipulation (20) pour la liaison mécanique à l'extrémité proximale (34) d'une tige (13), pour la formation d'un instrument médical (10), avec:
un dispositif de maintien (40, 50, 60) pour le maintien séparable de l'extrémité proximale (34) d'une tige (13) ou de l'extrémité proximale (35, 36) d'un dispositif de transmission pour la transmission au moins soit d'une force soit d'un couple de rotation;
un dispositif d'actionnement actionnable manuellement (70) ;
un dispositif de couplage (71, 72, 73, 75, 81, 85) pour le couplage mécanique du dispositif d'actionnement (70) au dispositif de maintien (40, 50, 60), de telle manière qu'un actionnement manuel du dispositif d'actionnement (70) provoque une séparation du dispositif de maintien (40, 50, 60),
dans lequel le dispositif de maintien (40, 50, 60) de concert avec une extrémité proximale (34, 35, 36) d'une tige (13) ou d'un dispositif de transmission maintenue par le dispositif de maintien (40, 50, 60) est déplaçable par rapport au dispositif d'actionnement (29) parallèlement à l'axe longitudinal (18) de la tige (13) ou du dispositif de transmission,
dans lequel le dispositif de couplage (71, 72, 73, 75, 81, 85) est configuré de façon à coupler le dispositif d'actionnement (70), indépendamment de la position du dispositif de maintien déplaçable (40, 50, 60) par rapport au dispositif d'actionnement (70), au dispositif de maintien déplaçable (40, 50, 60), de telle manière que le dispositif de maintien soit séparable,
**caractérisé en ce que**
il est prévu une multiplicité de dispositifs de maintien (40, 50, 60), dans lequel chacun individuellement de la multiplicité de dispositifs de maintien (40, 50, 60) est réalisé en vue du maintien séparable de l'extrémité proximale (34) d'une tige (13) ou de l'extrémité proximale (35, 36) d'un dispositif de transmission pour la transmission au moins soit d'une force soit d'un couple de rotation dans la tige (13);
le dispositif de couplage (71, 72, 73, 75, 81, 85) est réalisé en vue du couplage mécanique du dispositif d'actionnement (70) à la multiplicité de dispositifs de maintien (40, 50, 60), de telle manière qu'un actionnement manuel du dispositif d'actionnement (70) provoque une séparation de la multiplicité de dispositifs de maintien (40, 50, 60).

2. Dispositif de manipulation (20) selon la revendication précédente, dans lequel le dispositif de maintien déplaçable (40, 50, 60) de concert avec une extrémité proximale (34, 35, 36) d'une tige (13) ou d'un dispositif de transmission maintenue par le dispositif de maintien déplaçable (40, 50, 60) peut tourner par rapport au dispositif d'actionnement (70) autour d'un axe longitudinal (18) de la tige (13) ou du dispositif de transmission.

3. Dispositif de manipulation (20) selon la revendication précédente, dans lequel
un premier dispositif de maintien (40) de la multiplicité de dispositifs de maintien (40, 50, 60) est réalisé en vue de maintenir une extrémité proximale (34) d'une tige (13),
un second dispositif de maintien (50, 60) de la multiplicité de dispositifs de maintien (40, 50, 60) est réalisé en vue de maintenir une extrémité proximale (35, 36) d'un dispositif de transmission pour transmettre au moins soit une force soit un couple de rotation entre le dispositif de manipulation (20) et un outil (14) à l'extrémité distale de la tige (13).

4. Dispositif de manipulation (20) selon l'une quelconque des revendications précédentes, dans lequel le dispositif d'actionnement (70) est déplaçable manuellement pour l'actionnement dans la direction parallèle à l'axe longitudinal (18) d'une tige (13) reliée au dispositif de manipulation (20).

5. Dispositif de manipulation (20) selon l'une quelconque des revendications précédentes, dans lequel le dispositif de couplage présente:
un élément de couplage (70, 72, 73), qui est déplaçable dans une première direction de déplacement (77),
une face de glissement (71, 75) avec une région, qui n'est pas parallèle à la première direction de déplacement (77), pour convertir un déplacement de l'élément de couplage (70, 73) dans la première direction de déplacement (77) en un déplacement d'un verrou (41, 51, 61) dans une seconde direction de déplacement (87), qui est différente de la première direction de déplacement (77).

6. Dispositif de manipulation (20) selon la revendication précédente, dans lequel le verrou (61) est appliqué sur la face de glissement (71).

7. Dispositif de manipulation (20) selon la revendication 5, avec en outre un autre élément de couplage (81), qui est appliqué sur l'élément de couplage (73) et qui est déplaçable dans la seconde direction de déplacement (87), dans lequel le verrou (41, 51, 61) est couplé mécaniquement à l'autre élément de couplage (81).

8. Dispositif de manipulation (20) selon la revendication précédente, dans lequel soit l'autre élément de couplage (81) présente la face de glissement soit la face de glissement (75) est appliquée de façon glissante sur l'autre élément de couplage (81).

9. Dispositif de manipulation (20) selon la revendication 7 ou 8, en relation avec l'une quelconque des revendications 3 à 6, dans lequel l'autre élément de couplage (81) présente une face d'appui (86), qui est parallèle à l'axe longitudinal (18) d'une extrémité proximale (34, 35) d'une tige (13) ou d'un dispositif de transmission maintenue par le dispositif de maintien déplaçable (40, 50), et sur laquelle un verrou (41, 51, 61) du dispositif de maintien déplaçable (40, 50) est appliqué.

10. Dispositif de manipulation (20) selon l'une quelconque des revendications 5 à 9, dans lequel la seconde direction de déplacement (87) est au moins soit perpendiculaire à la première direction de déplacement (77) soit perpendiculaire à l'axe longitudinal (18) d'une tige (13) reliée au dispositif de manipulation (20).

11. Instrument médical (10) avec un dispositif de manipulation (20) selon l'une quelconque des revendications précédentes.
